**Europäisches Patentamt**

**European Patent Office**

**Office européen des brevets**

(19)

(11) Veröffentlichungsnummer : **0 464 460 B1**

(12)

# EUROPÄISCHE PATENTSCHRIFT

(45) Veröffentlichungstag der Patentschrift :
25.05.94 Patentblatt 94/21

(51) Int. Cl.$^5$ : **C07C 68/00, C07C 69/96**

(21) Anmeldenummer : **91110001.4**

(22) Anmeldetag : **19.06.91**

(54) **Verfahren zur Herstellung von Dialkylcarbonaten.**

(30) Priorität : **30.06.90 DE 4020941**

(43) Veröffentlichungstag der Anmeldung :
**08.01.92 Patentblatt 92/02**

(45) Bekanntmachung des Hinweises auf die
Patenterteilung :
**25.05.94 Patentblatt 94/21**

(84) Benannte Vertragsstaaten :
**BE CH DE FR GB IT LI NL**

(56) Entgegenhaltungen :
**EP-A- 0 425 197**
**GB-A- 2 003 872**

(73) Patentinhaber : **BAYER AG**
**D-51368 Leverkusen (DE)**

(72) Erfinder : **Klausener, Alexander, Dr.**
**Weissdornweg 37**
**W-5190 Stolberg (DE)**
Erfinder : **Landscheidt, Heinz, Dr.**
**Liliencronstrasse 6**
**W-4100 Duisburg (DE)**
Erfinder : **Blank, Heinz Ulrich, Dr.**
**Am Geusfelde 35**
**W-5068 Odenthal (DE)**
Erfinder : **Kipshagen, Walter, Dr.**
**Fichtestrasse 2a**
**W-5090 Leverkusen (DE)**

EP 0 464 460 B1

## Beschreibung

Gegenstand der Erfindung ist ein neues Verfahren zur Herstellung von Dialkylcarbonaten durch Umsetzung von Kohlenmonoxid mit Alkylnitriten in einer kontinuierlichen Gasphasenreaktion an einem Katalysator.

Dialkylcarbonate sind von allgemeiner chemischer Bedeutung. So ist beispielsweise Diethylcarbonat ein ausgezeichnetes Lösungsmittel im mittleren Siedebereich. Desweiteren sind die Dialkylcarbonate ausgezeichnete Carbonylierungs- und Alkylierungsreagenzien. Schließlich haben sie große Bedeutung bei der Herstellung von anderen Carbonaten, von Urethanen und Harnstoffen erlangt.

In der Literatur sind verschiedene Methoden zur Herstellung von Dialkylcarbonaten beschrieben. Von technisch großer Bedeutung sind dabei bis zum heutigen Tag Umsetzungen von Phosgen bzw. von Chlorameisensäurealkylestern mit Alkoholen (Ullmann's Encyclopedia of Industrial Chemistry, Vol. A 5 (1986), 197-202). Es besteht jedoch ein steigendes Interesse daran, den Einsatz des giftigen Phosgens bzw. der davon abgeleiteten Zwischenprodukte, wie der Chlorameisensäureester, durch andere Verfahren abzulösen. So ist die Herstellung von Dialkylcarbonaten durch Umsetzung von Kohlenmonoxid mit niederen Alkoholen in Gegenwart verschiedener Katalysatoren und Cokatalysatoren bekannt. Gemäß EP 365 083 werden niedere Alkohole mit Kohlenmonoxid in Gegenwart von Sauerstoff an einem Katalysatorsystem aus Kupferalkoxyhalogenid und Wasser umgesetzt. Nach EP 354 970 werden ebenfalls niedere Alkohole mit Kohlenmonoxid in Gegenwart von Sauerstoff an einem Katalysatorsystem umgesetzt, das aus einer Kombination eines Kupferkatalysators oder eines Platinmetall-Katalysators und einer Erdalkaliverbindung besteht, wobei sowohl das Kupfer bzw. das Platinmetall als auch das Erdalkalimetall als Salz einer schwachen Säure oder als Halogenid eingesetzt werden.

Aus JP 60-181 051 (1985) (C.A. 104 (1986, 110 355 t)) ist die Umsetzung von Alkylnitrit mit Kohlenmonoxid in der Gasphase an einem Platinmetall-Katalysator auf Trägern bekannt, wobei in Gegenwart von 10 Mol-%, bezogen auf CO, eines oxidierenden Mittels, beispielsweise Sauerstoff, gearbeitet wird. Es ist weiter aus EP 57 629 bekannt, daß in einem Gasphasenverfahren in Gegenwart eines Palladium-Trägerkatalysators Alkylnitrit, das in situ aus dem betreffenden Alkohol und einem Stickstoffoxid hergestellt wird, mit CO zu Dialkyloxalat umgesetzt wird.

Es bestand das Bestreben, ein kontinuierliches Gasphasen-Verfahren zur Herstellung von Dialkylcarbonaten zur Verfügung zu haben, in welchem ohne zusätzliche oxidierende Substanzen gearbeitet werden kann, die in jedem Fall in Gegenwart eines Katalysators bei erhöhter Temperatur zu einem zündfähigen Gemisch führen, welches unter sicherheitstechnischen Gesichtspunkten nicht in die großtechnische Produktion eingeführt werden kann. Ferner muß angenommen werden, daß die zusätzliche oxidierende Substanz einen beträchtlichen Teil des CO in unerwünschter Weise zu $CO_2$ umsetzt, was zu schwerwiegenden Ausbeuteverlusten führen muß.

Es wurde ein Verfahren zur Herstellung von Dialkylcarbonaten der Formel

$$O=C(OR)_2 \qquad (I),$$

worin

R geradkettiges oder verzweigtes $C_1$-$C_4$-Alkyl bedeutet,
durch Umsetzung von Kohlenmonoxid mit Alkylnitriten der Formel

$$RONO \qquad (II),$$

worin

R die oben angegebene Bedeutung hat,
in Gegenwart eines Inertgases an einem Platinmetall Träger-Katalysator bei erhöhter Temperatur in kontinuierlicher Gasphasenreaktion gefunden, welches dadurch gekennzeichnet ist, daß die Reaktion unter Ausschluß zusätzlicher oxidierender Substanzen und mit oder ohne zugesetztem Alkohol der Formel

$$ROH \qquad (III),$$

worin

R die angegebene Bedeutung hat,
durchgeführt wird, wobei ein Platinmetall-Träger-Katalysator eingesetzt wird, der durch eines oder mehrere Elemente aus der Gruppe Blei, Molybdän, Wolfram, Vanadium, Schwefel, Selen, Tellur, Gold, Antimon und Arsen in einer Menge von 0,01 bis 8 Gew.-%, bezogen auf das Gesamtgewicht des Katalysators, modifiziert ist.

Die Reaktion im erfindungsgemäßen Verfahren erfolgt im Sinne der nachfolgenden Reaktionsgleichung:

$$CO + 2\ RONO \rightarrow O=C(OR)_2 + 2\ NO,$$

Das erfindungsgemäße Verfahren ergibt hohe Umsätze des in die Reaktion eingebrachten Kohlenmonoxids (CO) sowie hohe Selektivitäten zu den gewünschten Dialkylcarbonaten, die bei Kenntnis der bekannten Gasphasenreaktionen nicht erwartet werden konnten, So wird nur ein geringer Teil des eingesetzten Kohlenmonoxids zum unerwünschten Kohlendioxid umgesetzt. Neben den Dialkylcarbonaten entstehen erfindungsgemäß als weitere Wertstoffe die korrespondierenden Dialkyloxalate. Die Summe der Dialkylcarbonate und

2

EP 0 464 460 B1

Dialkyloxalate ergibt eine im Vergleich zum Stand der Technik verbesserte chemische Ausbeute, bezogen auf umgesetztes CO.

Geradkettiges oder verzweigtes Alkyl mit bis zu 4 C-Atomen ist beispielsweise Methyl, Ethyl, Propyl, i-Propyl, Butyl, i-Butyl, in bevorzugter Weise die genannten n-Alkyle, in besonders bevorzugter Weise Methyl und Ethyl und ganz besonders bevorzugt Methyl.

Grundsätzlich ist es möglich von einem Gemisch verschiedener Alkylnitrite auszugehen, wobei jedoch auch ein Gemisch verschiedener Dialkylcarbonate und gegebenenfalls unsymmetrisch substituierter Dialkylcarbonate entstehen kann. Im Sinne einer einheitlichen Reaktion ist es daher bevorzugt, von nur einem Alkylnitrit auszugehen.

Das Alkylnitrit wird mit einem Inertgas verdünnt, welches dann auch die Funktion eines Trägergases übernimmt. Als Inertgas seien alle unter den Reaktionsbedingungen chemisch stabilen Gase genannt, bevorzugt Edelgase, Stickstoff und Kohlendioxid, besonders bevorzugt Argon, Stickstoff, Kohlendioxid, ganz besonders bevorzugt Stickstoff und Kohlendioxid. Die Menge des Trägergases beträgt etwa 20 bis 80 Vol.-%, bevorzugt 30 bis 70 Vol.-% des gesamten an den Katalysator gebrachten Gasgemisches.

Das Volumenverhältnis von CO zu Alkylnitrit beträgt 1:2-15, bevorzugt 1:2-12, besonders bevorzugt 1:2-10.

Das erfindungsgemäße Verfahren kann mit oder ohne zugesetzten Alkohol der Formel (III) durchgeführt werden. Für den Fall, daß mit einem zugesetzten Alkohol gearbeitet wird, beträgt dessen Volumenverhältnis zum Kohlenmonoxid 0,1-10:1, bevorzugt 0,2-5:1. Insbesondere bei der Herstellung von Dimethylcarbonat kann der Zusatz von Methanol zweckmäßig sein.

Das erfindungsgemäße Verfahren wird bei einer Temperatur von 80-200°C, bevorzugt 90-180°C, besonders bevorzugt 100-150°C und einem Druck von 0,8-8 bar, bevorzugt 1-3 bar, besonders bevorzugt 1-2,5 bar durchgeführt.

Als Katalysatoren kommen solche der Platinmetallgruppe, allein oder als Gemisch mehrerer von ihnen, auf einem Träger in Frage. Von den Metallen der Platingruppe seien bevorzugt genannt: Platin, Ruthenium, Rhodium, Iridium und Palladium; besonders bevorzugt ist Palladium. Die Konzentration der Edelmetalle auf dem Träger beträgt 0,01-5 Gew.-%, bevorzugt 0,05-3 Gew.-%, bezogen auf das Gesamtgewicht des Katalysators. Als Träger kommen alle auch sonst für Platinmetall-Katalysatoren gebräuchliche in Frage, beispielsweise: Aluminiumoxide, Spinelle, Silikate, Montmorillionite, Zeolithe, Aktivkohlen, Molekularsiebe, Diatomeenerden, Siliciumcarbid, Siliciumoxid und andere.

Die erfindungsgemäß einzusetzenden modifizierten Platinmetall-Träger-Katalysatoren enthalten weitere Zusätze an Metallen und/oder Halbmetallen undloder Nichtmetallen in einer Gesamtmenge von 0,01-8 Gew.-%, bezogen auf das Gesamtgewicht des Katalysators. Solche Zusätze sind Blei, Molybdän, Wolfram, Vanadium, Schwefel, Selen, Tellur, Gold, Antimon, Arsen und andere allein oder als Gemisch mehrerer von ihnen, in elementarer oder gebundener Form. Bevorzugt sind Blei, Selen, Antimon, Tellur und Schwefel, allein oder als Gemisch mehrerer von ihnen, als solche Zusätze, besonders bevorzugt in einer Gesamtmenge von 0,01-4 Gew.-%, bezogen auf das Gesamtgewicht des Katalysators. Als gebundene Form solcher zugesetzten Elemente kommt vor allem die oxidische oder die sulfidische Form in Frage.

Die Herstellung solcher modifizierter Platinmetall-Träger-Katalysatoren erfolgt nach Methoden, die dem Fachmann grundsätzlich bekannt sind. So kann einer der genannten Träger mit der Lösung einer oder mehrerer Platinmetallverbindung(en) sowie der Lösung eines oder mehrerer modifizierender Zusätze getränkt oder besprüht werden, Die Platinmetalle und Zusätze können gemeinsam oder getrennt appliziert werden. Nach jeder Tränkung schließt sich im allgemeinen eine Trocknung an; der Katalysator wird zum Schluß seiner Herstellung vielfach kalziniert. Verbindungen der Platinmetalle und der Zusätze sind beispielsweise die Chloride, Nitrate, Acetate, deren Ionen beim Kalzinieren vom Katalysator entfernt werden. Elemente in höheren Wertigkeitsstufen können beispielsweise als Ammoniumsalze (Ammoniummolybdat, -vanadat, -antimonat, -arsenat usw.) eingesetzt werden. Schwefel kann beispielsweise auch durch Auftränken von organischen Substanzen (Thiophen, Thioether usw.) appliziert werden.

Die Katalysatorbelastung beträgt 1-800 l CO, bevorzugt 10-500 l CO pro Stunde und pro g Edelmetall auf dem Katalysator.

Die Herstellung der erfindungsgemäß einzusetzenden Alkylnitrite erfolgt nach bekannten Verfahren, beispielsweise aus dem zugehörigen Alkohol und salpetriger Säure, die in situ aus einem Alkalinitrit und einer Mineralsäure (z.B. Schwefelsäure) gebildet wird.

Das im Verlaufe des erfindungsgemäßen Verfahrens gebildete Stickstoffmonoxid kann kontinuierlich mit Sauerstoff und neuem Alkohol zu Alkylnitrit regeneriert werden (DE-OS 38 34 065) und gemeinsam mit nicht umgesetztem Kohlenmonoxid und nicht umgesetztem Alkylnitrit recyclisiert werden.

3

Beispiel 1

Durch einen auf 125°C beheizten gläsernen Rohrreaktor (250 cm$^3$), der mit Raschig-Ringen und 20 g (25 ml) eines Pd-Katalysators (0,5 % Pd auf Al$_2$O$_3$, dotiert mit 0,2 % Schwefel) gefüllt wurde, leitete man mit einer Volumengeschwindigkeit von 25,5 l/h ein Gasgemisch, das aus 39 % Stickstoff, 18 % Methanol, 35 % Methylnitrit und 8 % Kohlenmonoxid bestand (Vol.-%). Das aus dem Reaktor austretende Gas wurde abgekühlt, die kondensierten Produkte wurden gesammelt und ebenso wie die gasförmigen nicht kondensierten Produkte bezüglich ihrer Zusammensetzung analysiert. Es wurde eine Selektivität von 24 % an Dimethylcarbonat (DMC) und von 71 % an Dimethyloxalat (DMO), bezogen auf umgesetztes CO, erreicht. Die Ausbeuten, bezogen auf eingesetztes CO, betrugen 13 % DMC und 40 % DMO. Die Raum-Zeit-Ausbeute (STY = Space Time Yield) an DMC betrug 43 g/l·h.

Beispiele 2 - 7

Durch einen auf die jeweilige Temperatur beheizten Rohrreaktor (250 cm$^3$), der mit Raschig-Ringen und der zum Einsatz kommenden Menge m des jeweiligen Katalysators gefüllt wurde, leitete man mit einem Volumenstrom von 20 l/h ein Gasgemisch, das aus 50 % Stickstoff, 22,5 % Methylnitrit, 22,5 % Methanol und 5 % Kohlenmonoxid bestand (Vol.-%). Das aus dem Reaktor austretende Gas wurde abgekühlt, die kondensierten Proben wurden gesammelt und bezüglich ihrer Zusammensetzung analysiert Die Ergebnisse sind in Tabelle 1 wiedergegeben.

Tabelle 1

| Bsp. | Katalysator | m [g] | Selektivität bez. auf CO-Umsatz | | Ausbeute bez. auf CO-Einsatz | | STY DMC [g/l.h] |
|---|---|---|---|---|---|---|---|
| | | | DMC [%] | DMO [%] | DMC [%] | DMO [%] | |
| 2 | 0,5% Pd+0,05%S auf Al$_2$O$_3$ | 20 | 43 | 51 | 17 | 20 | 26 |
| 3 | 0,5% Pd+0,2%S auf Al$_2$O$_3$ | 20 | 44 | 41 | 14 | 13 | 23 |
| 4 | 0,5% Pd+0,05%S auf Al$_2$O$_3$ | 10 | 40 | 53 | 14 | 19 | 22 |
| 5 | 0,5% Pd+0,2%S auf Al$_2$O$_3$ | 20 | 42 | 55 | 17 | 23 | 27 |
| 6 | 0,5% Pd+0,05%S auf Al$_2$O$_3$ | 5 | 33 | 63 | 11 | 21 | 70 |
| 7 | 0,5% Pd+0,2%S auf Al$_2$O$_3$ | 10 | 38 | 42 | 15 | 17 | 50 |

**Patentansprüche**

1. Verfahren zur Herstellung von Dialkylcarbonaten der Formel

$$O=C(OR)_2 ,$$

worin

R        geradkettiges oder verzweigtes $C_1$-$C_4$-Alkyl bedeutet,
durch Umsetzung von Kohlenmonoxid mit Alkylnitriten der Formel

RONO ,

worin

R        die angegebene Bedeutung hat,
in Gegenwart eines Inertgases an einem Platinmetall-Träger-Katalysator bei erhöhter Temperatur in kontinuierlicher Gasphasenreaktion, dadurch gekennzeichnet, daß die Reaktion unter Ausschluß Zusätzlicher oxidierender Substanzen und mit oder ohne Zugesetztem Alkohol der Formel

ROH ,

worin

R        die angegebene Bedeutung hat,
durchgeführt wird, wobei ein Platinmetall-Träger-Katalysator eingesetzt wird, der durch eines oder mehrere Elemente aus der Gruppe Blei, Molybdän, Wolfram, Vanadium, Schwefel, Selen, Tellur, Gold, Antimon und Arsen in einer Menge von 0,01 bis 8 Gew.-%, bezogen auf das Gesamtgewicht des Katalysators, modifiziert ist.

2.   Verfahren nach Anspruch 1, dadurch gekennzeichnet, daß Alkylnitrit bzw. Alkohol als Alkylgruppe ein n-Alkyl, bevorzugt Methyl oder Ethyl, besonders bevorzugt Methyl enthalten.

3.   Verfahren nach Anspruch 1, dadurch gekennzeichnet, daß als Inertgas ein Edelgas, Stickstoff oder Kohlendioxid, bevorzugt Argon, Stickstoff oder Kohlendioxid, besonders bevorzugt Stickstoff oder Kohlendioxid in einer Menge von 20 bis 80 Vol.-%, bevorzugt 30 bis 70 Vol.-%, des gesamten an den Katalysator geführten Gasgemisches eingesetzt werden.

4.   Verfahren nach Anspruch 1, dadurch gekennzeichnet, daß das Volumenverhältnis von Kohlenmonoxid zu Alkylnitrit 1:2-15, bevorzugt 1:2-12, besonders bevorzugt 1:2-10 beträgt.

5.   Verfahren nach Anspruch 1, dadurch gekennzeichnet, daß im Falle der Mitverwendung eines Alkohols, bevorzugt bei der Herstellung von Dimethylcarbonat, das Volumenverhältnis von Alkohol zu Kohlenmonoxid 0,1-10:1, bevorzugt 0,2-5:1 beträgt.

6.   Verfahren nach Anspruch 1, dadurch gekennzeichnet, daß bei einer Temperatur von 80-200°C, bevorzugt 90-180°C, besonders bevorzugt 100-150°C und einem Druck von 0,8-8 bar, bevorzugt 1-3 bar, besonders bevorzugt 1-2,5 bar gearbeitet wird.

7.   Verfahren nach Anspruch 1, dadurch gekennzeichnet, daß der Platinmetall-Katalysator eines oder mehrerer Elemente aus der Gruppe Palladium, Platin, Ruthenium, Rhodium und Iridium, bevorzugt Palladium und/oder Iridium in einer Menge von 0,1-5 Gew.-%, bezogen auf das Gesamtgewicht des Katalysators, enthält.

8.   Verfahren nach Anspruch 1, dadurch gekennzeichnet, daß der Platinmetall-Träger-Katalysator durch Zusatz eines oder mehrerer Elemente aus der Gruppe Blei, Selen, Antimon, Tellur und Schwefel modifiziert wird.

9.   Verfahren nach Anspruch 1, dadurch gekennzeichnet, daß die Modifizierungselemente in einer Gesamtmenge von 0,01 bis 4 Gew.-%, bezogen auf das Gesamtgewicht des Katalysators, eingesetzt werden.


**Claims**

1.   Process for preparing dialkyl carbonates of the formula

$$O{=}C(OR)_2,$$

where

R        is a straight-chain or branched $C_1$-$C_4$-alkyl,
by reaction of carbon monoxide with alkyl nitrites of the formula

RONO,

where

R        is as defined above,
in the presence of an inert gas over a supported platinum metal catalyst at elevated temperature in a continuous gas-phase reaction, characterized in that the reaction is carried out with exclusion of additional oxidizing substances and with or without added alcohol of the formula

$$ROH,$$

where

R      is as defined above,

using a supported platinum metal catalyst modified by one or more elements selected from the group consisting of lead, molybdenum, tungsten, vanadium, sulphur, selenium, tellurium, gold, antimony and arsenic in an amount from 0.01 to 8% by weight, based on the total weight of the catalyst.

2. Process according to Claim 1, characterized in that the alkyl of the alkyl nitrite and of the alcohol is an n-alkyl, preferably methyl or ethyl, particularly preferably methyl.

3. Process according to Claim 1, characterized in that the inert gas used is a noble gas, nitrogen or carbon dioxide, preferably argon, nitrogen or carbon dioxide, particularly preferably nitrogen or carbon dioxide, in an amount from 20 to 80% by volume, preferably from 30 to 70% by volume, of the total gas mixture passed over the catalyst.

4. Process according to Claim 1, characterized in that the volume ratio of carbon monoxide to alkyl nitrite is 1:2-15, preferably 1:2-12, particularly preferably 1:2-10.

5. Process according to Claim 1, characterized in that if an alcohol is used, preferably in the preparation of dimethyl carbonate, the volume ratio of alcohol to carbon monoxide is 0.1-10:1, preferably 0.2-5:1.

6. Process according to Claim 1, characterized in that the process is carried out at a temperature of 80-200°C, preferably 90-180°C, particularly preferably 100-150°C and a pressure of 0.8-8 bar, preferably 1-3 bar, particularly preferably 1-2.5 bar.

7. Process according to Claim 1, characterized in that the platinum metal catalyst contains one or more elements selected from the group consisting of palladium, platinum, ruthenium, rhodium and iridium, preferably palladium and/or iridium, in an amount of 0.1-5% by weight, based on the total weight of the catalyst.

8. Process according to Claim 1, characterized in that the supported platinum metal catalyst is modified by addition of one or more elements selected from the group consisting of lead, selenium, antimony, tellurium and sulphur.

9. Process according to Claim 1, characterized in that the modifying elements are used in a total amount from 0.01 to 4% by weight, based on the total weight of the catalyst.

**Revendications**

1. Procédé de préparation de carbonates de dialkyle de formule

$$O = C(OR)_2,$$

dans laquelle

R représente un groupe alkyle à chaîne droite ou ramifiée en $C_1$-$C_4$, par réaction de l'oxyde de carbone avec des nitrites d'alkyle de formule

$$RONO,$$

dans laquelle

R a les significations indiquées ci-desssus,

en présence d'un gaz inerte sur un catalyseur consistant en un métal du groupe du platine appliqué sur support, à température élevée, en continu et en phase gazeuse, caractérisé en ce que la réaction est exécutée en l'absence d'agent oxydant étranger et avec adjonction ou non d'un alcool de formule

$$ROH,$$

dans laquelle R a les significations indiquées ci-dessus,

avec utilisation d'un catalyseur consistant en un métal du groupe du platine appliqué sur un support, qui est modifé par un ou plusieurs éléments du groupe consistant en le plomb, le molybdène, le tungstène, le vanadium, le soufre, le sélénium, le tellure, l'or, l'antimoine et l'arsenic, en quantité de 0,01 à 8 % du poids total du catalyseur.

2. Procédé selon la revendication 1, caractérisé en ce que le nitrite d'alkyle et l'alcool contiennent en tant que groupe alkyle un groupe n-alkyle, de préférence méthyle ou éthyle, et tout sépcialement méthyle.

3. Procédé selon la revendication 1, caractérisé en ce que l'on utilise en tant que gaz inerte un gaz rare, l'azote ou le dioxyde de carbone, de préférence l'argon, l'azote ou le dioxyde de carbone, plus spécialement l'azote ou le dioxyde de carbone, en quantité de 20 à 80 %, plus spécialement de 30 à 70 % du volume du mélange gazeux total envoyé sur le catalyseur.

4. Procédé selon la revendication 1, caractérisé en ce que les proportions relatives en volume oxyde de carbone/nitrite d'alkyle sont de 1:2 à 15, de préférence de 1:2 à 12 et tout spécialement de 1:2 à 10.

5. Procédé selon la revendication 1, caractérisé en ce que, lorsqu'on utilise conjointement un alcool, en particulier pour la préparation du carbonate de diméthyle, les proportions relatives en volume entre l'alcool et l'oxyde de carbone sont de 0,1 à 10:1, de préférence de 0,2 à 5:1.

6. Procédé selon la revendication 1, caractérisé en ce que l'on opère à des températures de 80 à 200°C, de préférence de 90 à 180°C, plus spécialement de 100 à 150°C, sous une pression de 0,8 à 8 bar, de préférence de 1 à 3 bar et plus spécialement de 1 à 2,5 bar.

7. Procédé selon la revendication 1, caractérisé en ce que le catalyseur à base d'un métal du groupe du platine contient un ou plusieurs éléments du groupe consistant en le palladium, le platine, le rthénium, le rhodium et l'iridium, de préférence le palladium et/ou l'iridium, en quantité de 0,1 à 5 % du poids total du catalyseur.

8. Procédé selon la revendication 1, caractérisé en ce que le catalyseur consistant en un métal du groupe du platine appliqué sur support est modifié par addition d'un ou plusieurs éléments du groupe consistant en le plomb, le sélénium, l'antimoine, le tellure et le soufre.

9. Procédé selon la revendication 1, caractérisé en ce que les éléments modifiants sont mis en oeuvre en quantité totale de 0,01 à 4 % du poids total du catalyseur.